# EUROPEAN PATENT APPLICATION

(11) **EP 3 372 279 A1**
(43) Date of publication of application: **12.09.2018**
(21) Application number: 17160118.0
(22) Date of filing: 09.03.2017
(51) Int. Cl.: A61N 5/06

(54) **ENERGY TRANSFORMING DEVICE AND TANNING BED COMPRISING SUCH ENERGY TRANSFORMING DEVICE**

(71) Applicant: Wolff, Friedrich, 4125 Riehen (CH)
(72) Inventor: Wolff, Friedrich, 4125 Riehen (CH)
(74) Representative: Latscha Schöllhorn Partner AG

(57) **Abstract**

The present invention relates to an energy transforming device comprising a radiation source (2) arranged to emit electromagnetic waves in a field of propagation; a source irradiator (3ᵣ; 2_{f};) arranged to provide electromagnetic waves towards the radiation source (2), and a voltaic element (4) arranged to convert electromagnetic waves into electricity, wherein the voltaic element (4) is positioned in the field of propagation. The present invention also relates to a tanning bed (1) comprising such energy transformation device.

## Description

### Technical Field

The present invention relates to an energy transforming device according to the preamble of independent claim 1 and more particularly to an energy transforming device adapted to transform at least a portion of the radiant energy carried by electromagnetic waves into electricity. The present invention also relates to a tanning bed, or similar, comprising such an energy transformation device.

Such energy transforming device can be used for conversion of energy associated with emission of electromagnetic waves by a radiation source into electrical energy, so as to maximize the use of such electromagnetic radiation. In the context of the present invention, the electromagnetic radiation to be converted can take several forms, according to a frequency spectrum, as in radio waves, microwaves, infra-red radiation (e.g. heat), visible light, ultraviolet radiation; x-rays, gamma radiation etc. In particular, the present invention allows to optimize the use of energy of light, both visible and non-visible, generated by a radiation source, such as UV lamps, in a way that the most of such energy is effectively employed and does not get dispersed without being put to an advantageous use.

### Background Art

Normally, apparatuses comprising radiation sources which emit electromagnetic waves in order to propagate them to a target destination to be energized, such as to a body part of an apparatus user, are affected by a high degree of energy dispersion. Only a relatively small percentage of the energy originally radiated is effectively employed to produce a useful work or intended effect.

Analogously, energy dispersion affects indoor tanning apparatuses, such as tanning beds or booths, wherein special tanning lamps, in the form of tanning bulbs or of tanning tubes, emit ultraviolet light to produce a cosmetic tan of the skin of an user and/or to boost production of vitamin D in the body of such user and/or to generate an improvement of the user's mood. In fact, the amount of energy generated by the lamps which is not directly absorbed by the body of the user tends to remain ineffective and is scattered in the surrounding environment.

More in general, the electromagnetic energy emanated by artificial light sources is typically not entirely exploited to produce useful work. Therefore, the ratio of required energy (input) to usable energy (output) and, thus, the efficiency of respective systems typically are comparably low.

Therefore, there is a need for an energy transforming device allowing a more complete utilization of the energy associated with electromagnetic waves emitted by a radiation source. The is also a more particular need for a tanning bed which comprises such an energy transformation device, so that energy associated with the ultraviolet light emitted by tanning lamps is more efficiently put to use.

### Disclosure of the Invention

According to the invention this need is settled by an energy transforming device as it is defined by the features of independent claim 1, and by a tanning bed as it is defined by independent claim 18. Preferred embodiments are subject of the dependent claims.

In particular, the invention deals with an energy transforming device comprising a radiation source arranged to emit electromagnetic waves in a field of propagation. In the context of the present invention, the field of propagation is the overall travel path or travel space followed by the electromagnetic waves, whether diffracted, refracted, reflected or the like.

The energy transforming device according to the present invention further comprises a source irradiator. The source irradiator is arranged to provide electromagnetic waves towards the above mentioned radiation source. Moreover, the energy transforming device comprises a voltaic element arranged to convert electromagnetic waves or their energy into electricity. The voltaic element is positioned in the field of propagation of the radiation source.

Thanks to the provision of the voltaic element in the described configuration, it can be ensured that at least part of the energy associated with the electromagnetic waves originally emitted does not go dispersed in the environment (e.g. if not absorbed by a target body interposed in the field of propagation), but it is instead transferred to the voltaic element for further use.

The radiation source and the source irradiator of the energy transforming device according to the present invention cooperate in a way that the electromagnetic waves traveling therebetween are also directed to the voltaic element positioned in the field of propagation of such waves. By exposing the voltaic element to the ensuing radiation, the transformation into electric energy of at least a portion of the energy associated with the electromagnetic waves originally emitted by the radiation source and/or by the source irradiator is allowed. Thereby, it has been shown that such transformation and energy recuperation can be particularly efficient since the source irradiator provides electromagnetic waves towards the radiation source. As a consequence, an optimal and more complete use of the overall energy carried by the electromagnetic waves originally emitted can be obtained.

Preferably, the electromagnetic radiation can be light, particularly ultraviolet light, e.g. UVA and/or UVB. The voltaic element can be at least a photovoltaic panel making use of semiconductors exhibiting a photovoltaic effect.

The radiation source can be arranged to generate electromagnetic waves at an illuminance of at least about 2'000 Lux, preferably of at least about 10'000 Lux, more preferably of at least about 30'000 Lux and particularly of at least about 50'000 Lux. By adjusting the radiation source in such a way the energy recuperation of the device can be particularly high.

The energy transformation device can comprise more than one radiation source, for instance arranged in clusters according to a linear or a different pattern. Preferably, the energy transformation device is provided with three to twelve radiation sources.

Preferably, the radiation source comprises at least one light emitting diode, also referred to as LED. Such light emitting diode may be manufactured to emit radiation in well defined and adjustable bandwidth such as in the ultraviolet range. A sequence of such LEDs, for instance a linear array thereof, may be used to create an oblong pattern of the radiation source.

Preferably, especially when a use of the energy transforming device is envisaged in the proximity of an operator or directly for the benefit of a user, in order to avoid blinding the radiation source is mounted in a glare cladding. Such a glare cladding lets anyhow the electromagnetic waves of the radiation source be transmitted in the intended direction in the field of propagation. Light dispersion and associated loss of energy can be reduced.

In a possible embodiment of the energy transformation device according to the present invention, the source irradiator may comprise a reflector arranged to provide electromagnetic waves towards the radiation source by redirecting the electromagnetic waves of the radiation source towards the radiation source itself. In this configuration, the voltaic element is adapted to receive, reflected and/or non-reflected, at least part of the energy associated with the electromagnetic waves traveling from the radiation source to the reflector and back towards the radiation source, so as to transform it into electric energy.

Additionally or alternatively, the source irradiator may comprise at least a further radiation source adapted to emit electromagnetic waves in a further field of propagation. In this case, the field of propagation and the further field of propagation have a common zone, so that the respectively emitted electromagnetic waves travel along paths that at least partially overlap. Preferably, when the source irradiator comprises such a further radiation source, the radiation source is positioned in the further field of propagation and the further radiation source is positioned in the field of propagation.

In the configuration above described where the source irradiator comprises a further radiation source, the voltaic element can be positioned in the common zone induced by the field of propagation and by the further field of propagation. Such an arrangement allows for a particular efficient energy recuperation.

In a particular efficient embodiment, the radiation source and the further radiation source may generate electromagnetic waves having wavelengths in an essentially identical range of wavelengths. Preferably, the essentially identical range of wavelengths is from about 320 nanometers to about 650 nanometers.

More specifically, the radiation source and the further radiation source may be of identical type.

Preferably, the radiation source is elastically supported. Additionally or alternatively, the voltaic element can be elastically supported. More particularly, the radiation source and/or voltaic element can be affixed or mounted in the energy transforming device in a way that it is elastically supported. Such elastic support allows the radiation source and/or voltaic element to vibrate which may be crucial in operation of the device. Also, it allows for better absorbing possible mechanical shocks such that a more durable and shock resistant structure can be created.

Preferably, the voltaic element has a power capability of at least 150 Watt. Even more preferably, the voltaic element is provided with a power capability of at least about 500 Watt. Such voltaic element allows for an efficient energy recuperation in connection with electromagnetic waves particularly light.

Preferably, the energy transformation device comprises a cooling arrangement, adapted to lower a temperature of the radiation source. Such feature depends on the nature of the source of radiation used. This is especially advantageous when an application of the device is contemplated that implies proximity or contact with a user or an operator, such as in the case of a tanning bed as below introduced. Also, it allows for efficiently operating the radiation source(s) over a comparably long period.

The present invention also relates to a tanning bed, or similar, comprising an energy transformation device according to the previous description.

Preferably, the tanning bad comprises at least a tanning lamp, wherein the tanning lamp is the radiation source of the energy transformation device above described.

A tanning bed according to the present invention can take the form of a substantially horizontal enclosure with an underlying bench and a cover, or lid, which house UV-emitting radiation sources such as bulbs or tubes, under an acrylic surface. In this configuration, the user, or tanner, is surrounded by the radiation sources when the cover is closed.

Alternatively, a tanning bed according to the present invention can take the form of a stand-up booth comprising a substantially vertical enclosure, wherein the tanner, surrounded by UV-emitting radiation sources, stands during exposure, for instance holding onto straps or handrails. A door, closable by the tanner, may be provided, or the booth may have an open design.

A tanning bed modified to incorporate an energy transformation device as described, allows to maximize exploitation of the energy radiated by tanning lamps, so that even the energy not directly concentrated on, and absorbed by, the body of a user or tanner is advantageously employed, preferably for operating the tanning bed itself or parts thereof. Thanks to the provision of a voltaic element, at least a portion of the tanning lamp light radiation is converted into electric power.

Preferably, the voltaic element of the energy transformation device is connected to the mentioned at least a tanning lamp, for operating the tanning lamp. The voltaic element can as well be operatively connected to other components of the tanning bed and can supply the energy necessary to control them accordingly.

Preferably, the tanning bed comprises an energy supply unit, connected to the tanning lamp, for operating the tanning lamp. Thanks to the control exerted by the energy supply unit, the tanning bed and the related lamp can be energized according to the current needs and consumption. The energy supply unit preferably manages the distribution of electric current coming from the voltaic element. The energy supply unit may also manage power drawn from an electric power mains. Therefore, an economical adjustment of the energy employed for the tanning lamps and in general for operating the tanning bed can be achieved, by sourcing the energy from the voltaic element and/or from an electric power mains.

Excess energy converted by the voltaic element which is not immediately employed can be stored, for successive use, in a power storage unit, operatively connected to the voltaic element and to the energy supply unit. Thus, a power module may be created - via an electronic circuitry interconnecting voltaic element, power storage unit and energy supply unit - to effectively convey photovoltaic-sourced electrical energy to the tanning lamp of the tanning bed, or to a multiplicity of tanning lamps thereof. As explained, the amount and the duration of provision of such photovoltaic-sourced electrical energy for the functioning of the tanning bed itself can be adjusted according to real-time needs.

The electricity resulting from the photovoltaic conversion by the voltaic element may be initially direct current electricity. Therefore, the tanning bed according to the present invention preferably comprises a power inverter circuitry, such as a DC/AC power converter; voltage regulators and the like.

### Brief Description of the Drawings

The energy transforming device according to the invention, as well as a tanning bed comprising such an energy transforming device, are described in more detail herein below by way of exemplary embodiments and with reference to the attached drawings, in which:
- Fig. 1: shows a perspective view of a first embodiment of a tanning bed according to the present invention;
- Fig. 2: shows a section view of a second embodiment of a tanning bed according to the present invention;
- Fig. 3: shows a bottom view of a cover of a tanning bed according to the present invention;
- Fig. 4: shows a perspective view of a quadrangular module incorporating an energy transforming device according to the present invention;
- Fig. 5: shows a top view of the quadrangular module of Figure 4; and
- Fig. 6: shows a perspective view of a triangular module incorporating an energy transforming device according to the present invention.

### Description of Embodiments

In the following description certain terms are used for reasons of convenience and are not intended to limit the invention. The terms "right", "left", "up", "down", "under" and "above" refer to directions in the figures. The terminology comprises the explicitly mentioned terms as well as their derivations and terms with a similar meaning. Also, spatially relative terms, such as "beneath", "below", "lower", "above", "upper", "proximal", "distal", and the like, may be used to describe one element's or feature's relationship to another element or feature as illustrated in the figures. These spatially relative terms are intended to encompass different positions and orientations of the devices in use or operation in addition to the position and orientation shown in the figures. For example, if a device in the figures is turned over, elements described as "below" or "beneath" other elements or features would then be "above" or "over" the other elements or features. Thus, the exemplary term "below" can encompass both positions and orientations of above and below. The devices may be otherwise oriented (rotated 90 degrees or at other orientations), and the spatially relative descriptors used herein interpreted accordingly. Likewise, descriptions of movement along and around various axes include various special device positions and orientations.

To avoid repetition in the figures and the descriptions of the various aspects and illustrative embodiments, it should be understood that many features are common to many aspects and embodiments. Omission of an aspect from a description or figure does not imply that the aspect is missing from embodiments that incorporate that aspect. Instead, the aspect may have been omitted for clarity and to avoid prolix description. In this context, the following applies to the rest of this description: If, in order to clarify the drawings, a figure contains reference signs which are not explained in the directly associated part of the description, then it is referred to previous or following description sections. Further, for reason of lucidity, if in a drawing not all features of a part are provided with reference signs it is referred to other drawings showing the same part. Like numbers in two or more figures represent the same or similar elements.

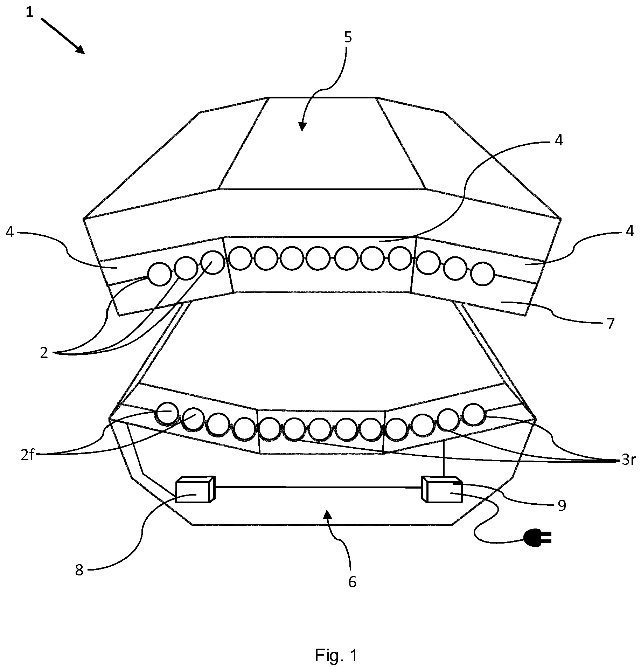

Fig. 1 shows a first embodiment of a tanning bed 1 which is especially modified to incorporate a first embodiment of a energy transforming device according to the present invention.

The tanning bed 1 comprises a cover 5 and a bench 6 on which the cover 5 closes to envelope a tanning bed user. The cover 5 supports, for instance by elastic means, a plurality of radiation sources in the form of tanning lamps 2, arranged to emit UV electromagnetic radiation. The field of propagation is mainly defined by the disposition of the lamps 2 within the space of the tanning bed 1 and by their orientation.

The plurality of tanning lamps 2 is disposed between a transparent spacer 7, for instance made of acrylic material, and a series of voltaic elements 4. The transparent spacer 7 and the voltaic elements 4 are also affixed to the cover 5. In the present specific case, there are three voltaic elements 4, arranged adjacent to each other and following the profile of the cover 5. The bench 6 supports a multiplicity of source irradiators. Such source irradiators are arranged to provide electromagnetic waves towards the tanning lamps 2. In the present first embodiment, the source irradiators comprise further radiation sources in the form of tanning lamps 2_{f} as well as glare claddings 3ᵣ. Glare claddings 3ᵣ are represented as separate elements, but can also be achieved by coating the inner tubular walls of the tanning lamps 2_{f} with a reflective material, by a given percentage of the circumferential surface of such walls. Tanning lamps 2_{f} are adapted to emit electromagnetic waves in a further field of propagation. The field of propagation of the electromagnetic waves originating from the tanning lamps 2 and the further filed of propagation have a common zone. The glare claddings 3ᵣ associated with tanning lamps 2_{f} redirect electromagnetic waves towards the tanning lamps 2, namely redirecting all electromagnetic waves impacting on them (i.e. waves both emitted from remote by the tanning lamps 2 and proximally emitted by the tanning lamps 2_{f}) towards the tanning lamps 2.

Other, different reflectors can also be provided to cooperate with the tanning lamps 2, in order to better define the relative field of propagation. In their absence, anyhow, at least a portion of the electromagnetic waves propagating from the tanning lamps 2 will be directly absorbed and converted into electric energy by the voltaic elements 4.

Thanks to the above configuration, the voltaic element 4 is positioned in the field of propagation of the electromagnetic waves and is configured to absorb the energy they carry and advantageously convert it into electricity. By field of propagation, as previously explained, the overall travel path or space followed by the electromagnetic waves is meant, also when reflected.

Thus, the radiation energy is harvested which, differently, would be dispersed in the environment if unabsorbed by the body of a tanning bed user.

Electricity converted by the voltaic element 4 can be stored in a power storage unit 8, readily available to supply energy to the tanning lamps 2, 2_{f} and/or to other components or functions of the tanning bed 1. The allocation of such energy is controlled by an energy supply unit 9, which can balance energy drawn from mains electricity with that derived from the voltaic element 4 for the functioning of the tanning bed 1.

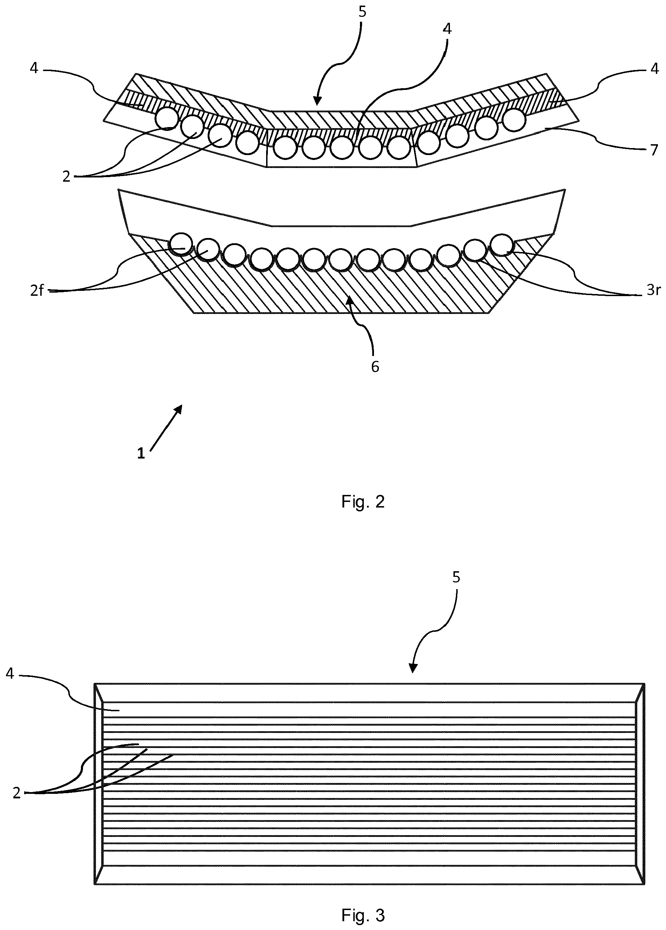

Fig. 2 shows a second embodiment of a tanning bed 1 according to the present invention. Whereas the design of such second embodiment is different, particularly with respect to shape of cover 5 and bench 6 which are in this case concave rather than convex, the functioning remains substantially the same as for the first embodiment. The section view enhances visibility of radiation sources 2, voltaic elements 4, and source irradiators 3ᵣ, 2_{f}.

Fig. 3 shows a bottom view of a cover 5 of a tanning bed 1 according to the present invention. Through the transparent spacer 7, a multiplicity of radiation sources in the form of elongated tanning lamps 2, or tubes, is visible. The tanning lamps 2 appear disposed between the transparent spacer 7 and a voltaic element 4.

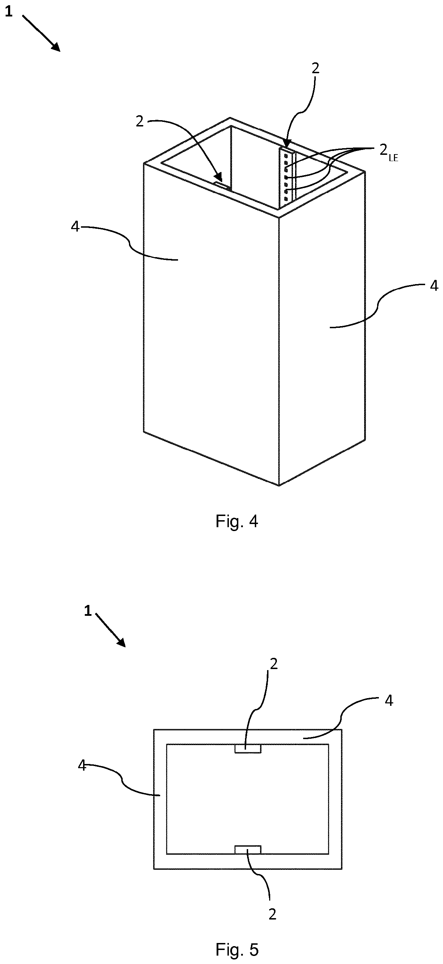

Figs. 4 and 5 show a quadrangular module 10 incorporating a second embodiment of an energy transforming device according to the present invention. Four voltaic elements 4 in the form of panels are arranged to form a substantially rectangular or cuboid hollow section or space.

A radiation source 2 is affixed to an internal wall of one of the larger voltaic elements 4. On the internal wall of the voltaic element opposite to that, a source irradiator 2_{f} is positioned to provide electromagnetic waves towards the radiation source 2. The radiation source 2 comprises a linear array of light emitting diodes 2_{LED}. The source irradiator comprises a further radiation source 2_{f} which similarly comprises an array of LEDs. The radiation source 2 and the further radiation source 2_{f} emit electromagnetic waves which travel along respective fields of propagation. Given the arrangement exemplified in Figure 5, the radiation source is positioned in the further field of propagation of the further radiation source 2_{f}, and vice-versa. The four voltaic elements 4 are positioned in the fields of propagation, and at least the smaller lateral voltaic elements are in a common zone thereof.

The boxlike, laterally closed structure of the quadrangular module 10 allows for beneficial collection by the voltaic elements 4 of any energy carried by the electromagnetic waves which remains available because, for instance, unabsorbed by a body placed inside the module 10. Such energy can be transformed by the voltaic elements 4 in electric current.

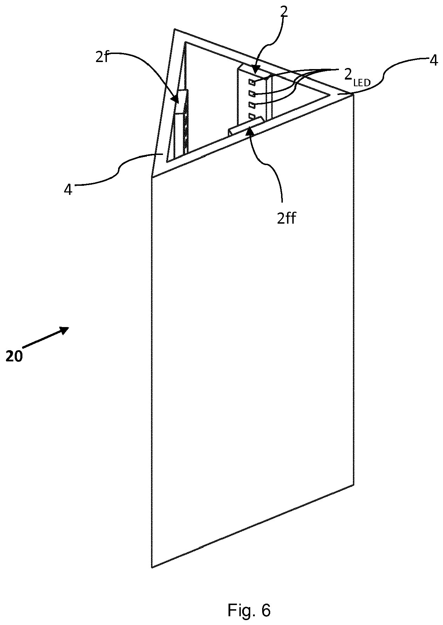

Figure 6 shows a perspective view of a triangular module 20 incorporating a third embodiment of an energy transforming device according to the present invention. A radiation source 2 is affixed to an internal wall of one of the three voltaic elements 4. On the internal wall of the other two voltaic elements, respectively a source irradiator 2_{f} and an additional source irradiator 2_{ff} -both in the form of further radiation sources- are positioned to provide electromagnetic waves towards the radiation source 2 and towards each other. Mutatis mutandis, the functioning of the triangular module 20 is similar to what explained in connection with the quadrangular module 10. The energy correlated to the emitted electromagnetic waves is optimally harnessed within the triangular module 20 and a portion thereof is advantageously converted into electric energy via the voltaic elements 4.

The radiation source 2 and the further radiation source 2_{f}, 2_{ff} may be of identical type; also, they may generate electronic waves with an essentially identical range of wavelengths.

This description and the accompanying drawings that illustrate aspects and embodiments of the present invention should not be taken as limiting the claims defining the protected invention. In other words, while the invention has been illustrated and described in detail in the drawings and foregoing description, such illustration and description are to be considered illustrative or exemplary and not restrictive. Various mechanical, compositional, structural, electrical, and operational changes may be made without departing from the spirit and scope of this description and the claims. In some instances, well-known circuits, structures and techniques have not been shown in detail in order not to obscure the invention. Thus, it will be understood that changes and modifications may be made by those of ordinary skill within the scope and spirit of the following claims. In particular, the present invention covers further embodiments with any combination of features from different embodiments described above and below.

The disclosure also covers all further features shown in the Figs. individually although they may not have been described in the afore or following description. Also, single alternatives of the embodiments described in the figures and the description and single alternatives of features thereof can be disclaimed from the subject matter of the invention or from disclosed subject matter. The disclosure comprises subject matter consisting of the features defined in the claims or the exemplary embodiments as well as subject matter comprising said features.

Furthermore, in the claims the word "comprising" does not exclude other elements or steps, and the indefinite article "a" or "an" does not exclude a plurality. A single unit or step may fulfil the functions of several features recited in the claims. The mere fact that certain measures are recited in mutually different dependent claims does not indicate that a combination of these measures cannot be used to advantage. The terms "essentially", "about", "approximately" and the like in connection with an attribute or a value particularly also define exactly the attribute or exactly the value, respectively. The term "about" in the context of a given numerate value or range refers to a value or range that is, e.g., within 20%, within 10%, within 5%, or within 2% of the given value or range. Components described as coupled or connected may be electrically or mechanically directly coupled, or they may be indirectly coupled via one or more intermediate components. Any reference signs in the claims should not be construed as limiting the scope.

## Claims

1. Energy transforming device comprising
a radiation source (2) arranged to emit electromagnetic waves in a field of propagation,
a source irradiator (3ᵣ; 2_{f}; 2_{ff}) arranged to provide electromagnetic waves towards the radiation source (2), and
a voltaic element (4) arranged to convert electromagnetic waves into electricity, wherein
the voltaic element (4) is positioned in the field of propagation.

2. Energy transformation device according to claim 1, wherein the radiation source (2) is arranged to generate electromagnetic waves at an illuminance of at least about 2'000 Lux, preferably of at least about 10'000 Lux, more preferably of at least about 30'000 Lux and particularly of at least about 50'000 Lux.

3. Energy transformation device according to claim 1 or 2, wherein the radiation source (2) is elastically supported.

4. Energy transformation device according to any one of the preceding claims, wherein the voltaic element (4) is elastically supported.

5. Energy transformation device according to any one of the preceding claims, wherein the radiation source (2) is mounted in a glare cladding.

6. Energy transformation device according to any one of the preceding claims, wherein the source irradiator comprises a reflector (3ᵣ) arranged to provide electromagnetic waves towards the radiation source (2) by redirecting the electromagnetic waves of the radiation source (2) towards the radiation source (2).

7. Energy transformation device according to any one of the preceding claims, wherein the source irradiator comprises a further radiation source (2_{f}, 2_{ff}) adapted to emit electromagnetic waves in a further field of propagation, wherein the field of propagation and the further field of propagation have a common zone.

8. Energy transformation device according to claim 7, wherein the voltaic element (4) is positioned in the common zone.

9. Energy transforming device according to claim 7 or 8, wherein the radiation source (2) is positioned in the further field of propagation and the further radiation source (2_{f}, 2_{ff}) is positioned in the field of propagation.

10. Energy transformation device according to any one of claims 7 to 9, wherein the radiation source (2) and the further radiation source (2_{f}, 2_{ff}) generate electromagnetic waves having wavelengths in an essentially identical range of wavelengths.

11. Energy transformation device according to any one of the preceding claims, comprising three to twelve radiation sources (2, 2_{f}, 2_{ff}).

12. Energy transformation device according to any one of the preceding claims, wherein the voltaic element comprises a photovoltaic panel (4).

13. Tanning bed (1) comprising an energy transformation device according to any on of the preceding claims or to any one of claims 16 to 20.

14. Tanning bed (1) according claim 13, comprising a tanning lamp (2), wherein the tanning lamp (2) is the radiation source of the energy transformation device.

15. Tanning bed (1) according to claim 14, wherein the voltaic element (4) of the energy transformation device is connected to the tanning lamp (2) for operating the tanning lamp (2).

16. Energy transformation device according to any one of claims 1 to 13, wherein the radiation source (2) comprises at least one light emitting diode (2_{LED}).

17. Energy transforming device according to any one of claims 10 to 12 or 16, wherein the essentially identical range of wavelengths is from about 320 nanometers to about 650 nanometers.

18. Energy transformation device according to any one of claims 7 to 12 or 16 or 17, wherein the radiation source (2) and the further radiation source (2_{f}, 2_{ff}) are of identical type.

19. Energy transformation device according to any one of claims 1 to 13 or 16 to 18, comprising a cooling arrangement adapted to lower a temperature of the radiation source (2).

20. Energy transformation device according to any one of claims 1 to 13 or 16 to 19, wherein the voltaic element (4) has a power capability of at least 150 Watt and preferably of at least about 500 Watt.

21. Tanning bed (1) according to claim according to any one of claims 13 to 15, comprising an energy supply unit (9) connected to the tanning lamp (2) for operating the tanning lamp (2).

22. Tanning bed (1) according to claim 21, comprising a power storage unit (8) operatively connected to the voltaic element (4) and to the energy supply unit (9), for storing electric energy converted by the voltaic element (4).
